# EUROPEAN PATENT APPLICATION

(11) **EP 3 603 488 A1**
(43) Date of publication of application: **05.02.2020**
(21) Application number: 18187224.3
(22) Date of filing: 03.08.2018
(51) Int. Cl.: A61B 5/00, A61B 7/00, A61B 5/053

(54) **APPARATUS AND METHOD FOR DISPLAYING PATIENT PAIN INFORMATION**

(71) Applicant: Koninklijke Philips N.V., 5656 AE Eindhoven (NL)
(72) Inventor: Vuppala, Sunil Kumar, 5656 AE Eindhoven (NL); Sisodia, Rajendra Singh, 5656 AE Eindhoven (NL); Bhat, Ravindra, 5656 AE Eindhoven (NL); Johnson, Mark Thomas, 5656 AE Eindhoven (NL); Helle, Michael Günter, 5656 AE Eindhoven (NL); Weiss, Steffen, 5656 AE Eindhoven (NL)
(74) Representative: de Haan, Poul Erik

(57) **Abstract**

The present invention relates to an apparatus (10) for displaying patient pain information. The apparatus comprises a sheet (20), an input unit (30), a processing unit (40), and at least one light source (50). The sheet is configured to support a patient during transfer from a bed of the patient to a bed of a diagnostic imaging system. The input unit is configured to receive information relating to at least one known pain area of at least one part of the patient's body. The input unit is configured to provide the processing unit with the information relating to the at least one known pain area of at least one part of the patient's body. The processing unit is configured to control the at least one of the at least one light source to provide light illumination at at least one first position of the sheet. Control of the at least one of the at least one light source comprises utilization of the information relating to the at least one known pain area of at least one part of the patient's body.

## Description

### FIELD OF THE INVENTION

The present invention relates to an apparatus for displaying patient pain information and to a method for displaying patient pain information.

### BACKGROUND OF THE INVENTION

The workflow for diagnostic imaging exams with CT, MR, PET and hybrid systems includes the transfer of patients, who may be unable to communicate effectively, from the hospital bed to the scan bed before the exam and back after the exam. In current standard clinical practice, several staff members lift and pull the patient manually from the patient's bed to the scan bed with the help of a bed sheet. The identification of pain areas of the patient is important during this transfer, and helps minimize movement of the patient during the scan. Thus, both the pain areas and position are important to be ready for the scan. However, this identification can be difficult with patients who cannot express their pain areas verbally.

There is a need to address these issues.

### SUMMARY OF THE INVENTION

It would be advantageous to have improved means to identify pain areas of a patient when transferring them to a scanner system.

The object of the present invention is solved with the subject matter of the independent claims, wherein further embodiments are incorporated in the dependent claims. It should be noted that the following described aspects and examples of the invention apply also to the apparatus for displaying patient information and to the method for displaying patient information.

According to a first aspect, there is provided an apparatus for displaying patient pain information, comprising:
- a sheet;
- an input unit;
- a processing unit; and
- at least one light source.

The sheet is configured to support a patient during transfer from a bed of the patient to a bed of a diagnostic imaging system. The input unit is configured to receive information relating to at least one known pain area of at least one part of the patient's body. The input unit is configured also to provide the processing unit with the information relating to the at least one known pain area of at least one part of the patient's body. The processing unit is configured to control the at least one of the at least one light source to provide light illumination at at least one first position of the sheet. Control of the at least one of the at least one light source comprises utilization of the information relating to the at least one known pain area of at least one part of the patient's body.

In this manner, patients can be moved and examined with diagnostic imaging systems, where those patients may not be able express if they are in pain, but medical staff are clearly provided with information as to where the patient has pain. Furthermore, a staff member may know about where a particular patient is feeling pain, but they may not be with that patient when they are being moved and/or examined elsewhere. However, information from this informed staff member can be used to provide appropriate illumination of the sheet on which the patient is lying in order that they are than duly informed. Thus, the apparatus has particular utility to those patients who may be handicapped in a manner that makes it difficult for them to express their pain to other quickly and clearly, or for children etc.

In an example, the at least one light source is integrated into the sheet.

In an example, the apparatus comprises at least one pressure sensor integrated into the sheet. The at least one pressure sensor is configured to provide the processing unit with pressure related information resulting from the patient's body lying on the sheet.

In an example, the processing unit is configured to utilize the pressure related information to control the at least one of the at least one light source to provide light illumination at the at least one first position of the sheet.

In this manner, it is known what parts of the patient have pain and pressure sensors can be used to locate the patient on the sheet, and then the sheet can be illuminated by integrated light sources in the sheet at the correct positons of the patient.

In an example, the processing unit is configured to utilize the pressure related information to control one or more of the at least one light source to provide light illumination at at least one second position of the sheet.

In an example, the processing unit is configured to process the pressure related information to determine a change in pressure at a location of the sheet associated with a part of the patient's body when lying on the sheet. The processing unit is configured also to control the one or more of the at least one light source to provide light illumination at the location of the sheet where there was a pressure change.

In other words, if the patient for example releases a part of their body from the sheet, a change in pressure is detected and the sheet at that location can be appropriately illuminated in order that medical staff can be made aware and take remedial action.

In an example, the processing unit is configured to control the at least one of the at least one light source to provide light at the at least one first position in a first colour and to control the one or more of the at least one light source to provide light at the at least one second position in a second colour.

In an example, all of the input unit, the processing unit and the at least one light source are separate from the sheet.

In this way, a Magnetic Resonance compatible smart sheet is provided as part of the apparatus.

In an example, the apparatus comprises at least one pain sensing device. The at least one pain sensing device is configured to provide the processing unit with information relating to the patient. The processing unit is configured to control the at least one light source on the basis of the pain information.

In an example, the at least one pain sensing device comprises a galvanic skin conductance sensor. The information relating to the patient can then comprise a skin conductance of the patient.

In this manner, for an episode of acute pain about 1 second after this commences there is a large increase in the patient's skin conductance. The processing unit can then for example set all the at least one light source to illuminate the entire sheet to inform medical staff that immediate attention is required.

In an example, the galvanic skin conductance sensor is at least partially integrated into the sheet.

In an example, the at least one pain sensing device comprises a camera configured to acquire imagery to the patient. The information relating to the patient can then comprise imagery of the face of the patient and/or of the body of the patient.

In this manner, if a facial expression of the patient and/or cramping or spasm is detected, the processing unit can then for example set all the at least one light source to illuminate the entire sheet to inform medical staff that immediate attention is required.

In an example, the at least one pain sensing device comprises a microphone. The information relating to the patient can then comprise one or more sounds associated with the patient.

In this manner, if the processing unit determines that the patient has cried out or otherwise expressed that they are in pain verbally, the processing unit can then for example set all the at least one light source to illuminate the entire sheet to inform medical staff that immediate attention is required.

In an example, the microphone is integrated into the sheet.

According to a second aspect, there is provided a method for displaying patient pain information, comprising:
a) using an input unit to input information relating to at least one known pain area of at least one part of a patient's body;
b) providing a processing unit with the information relating to the at least one known pain area of at least one part of the patient's body;
c) laying a patient on a sheet, wherein the sheet is configured to support a patient during transfer from a bed of the patient to a bed of a diagnostic imaging system;
d) controlling by the processing unit at least one of at least one light source to provide light illumination at at least one first position of the sheet, wherein control of the at least one of the at last one light source comprises utilization of the information relating to the at least one known pain area of at least one part of the patient's body.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an apparatus for displaying patient information;
Fig. 2 shows a method for displaying patient information; and
Fig. 3 shows a schematic illustration of a bed sheet forming part of the apparatus.

### DETAILED DESCRIPTION OF THE EMBODIMENTS

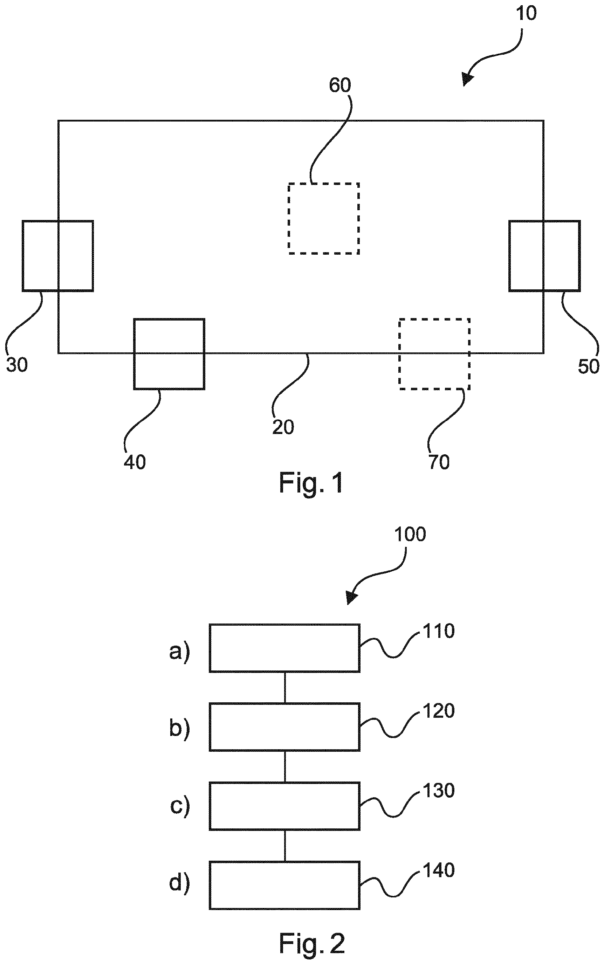

Fig. 1 shows an example of an apparatus 10 for displaying patient pain information. The apparatus 10 comprises a sheet 20, an input unit 30, a processing unit 40, and at least one light source 50. The sheet is configured to support a patient during transfer from a bed of the patient to a bed of a diagnostic imaging system. The input unit is configured to receive information relating to at least one known pain area of at least one part of the patient's body. The input unit is configured also to provide the processing unit with the information relating to the at least one known pain area of at least one part of the patient's body. The processing unit is configured to control the at least one of the at least one light source to provide light illumination at at least one first position of the sheet. Control of the at least one of the at least one light source comprises utilization of the information relating to the at least one known pain area of at least one part of the patient's body.

In an example, the at least one light source is at least one LED, that can operate at different colours.

In an example, the information received by the input unit was input by a user.

In an example, the information received by the input unit was provided by a machine - for example the output from an electronic health record, or the output of an AI decision support algorithm (e.g. CDS tool).

According to an example, the at least one light source is integrated into the sheet.

According to an example, the apparatus comprises at least one pressure sensor 60 integrated into the sheet. The at least one pressure sensor is configured to provide the processing unit with pressure related information resulting from the patient's body lying on the sheet.

According to an example, the processing unit is configured to utilize the pressure related information to control the at least one of the at least one light source to provide light illumination at the at least one first position of the sheet.

According to an example, the processing unit is configured to utilize the pressure related information to control one or more of the at least one light source to provide light illumination at at least one second position of the sheet.

According to an example, the processing unit is configured to process the pressure related information to determine a change in pressure at a location of the sheet associated with a part of the patient's body when lying on the sheet. The processing unit is configured to control the one or more of the at least one light source to provide light illumination at the location of the sheet where there was a pressure change.

In an example, the at least one first position comprises the at least one second positon. In other words, it is a known area of pain that would appear to be particularly painful for the patient.

In an example, the at least one second position is not comprised within the at least one first position. In other words, the patient would appear to be feeling pain at a part of the body that it was not known to be painful for the body. Thus, a new painful area for the patient can be detected and easily and effectively communicated to medical staff, who can then act as required.

In an example, the processing unit is configured to process the pressure related information to determine a change in pressure from one location to a second location of the sheet associated with a part of the patient's body when lying on the sheet; and wherein the processing unit is configured to control the one or more of the at least one light source to provide light illumination at the second location relative to the first position of the sheet.

In other words, when a patient shifts the location on the sheet from a position relative to another position, the pain information presented to medical practitioners also correspondingly changes, through the processing unit changing the light source at the relative locations. The relative location be again being derived from the change in the pressure.

According to an example, the processing unit is configured to control the at least one of the at least one light source to provide light at the at least one first position in a first colour and to control the one or more of the at least one light source to provide light at the at least one second position in a second colour.

According to an example, all of the input unit, the processing unit and the at least one light source are separate from the sheet.

In an example, the light is projected onto the sheet.

In an example, the light is brought into the sheet through a series of optical fibers from a central lighting unit (which can be at a distance from the sheet - e.g. outside the MRI). The fibers are then distributed across the sheet and terminate (and out-couple the light) at grid points in e.g. a square grid. In an example, there are more fibers close to the centre of the sheet. In this way there is higher spatial resolution where the patient will mostly lie.

According to an example, the apparatus comprises at least one pain sensing device 70. The at least one pain sensing device is configured to provide the processing unit with information relating to the patient. The processing unit is configured to control the at least one light source on the basis of the pain information.

According to an example, the at least one pain sensing device comprises a galvanic skin conductance sensor. The information relating to the patient can then comprise a skin conductance of the patient.

According to an example, the galvanic skin conductance sensor is at least partially integrated into the sheet.

According to an example, the at least one pain sensing device comprises a camera configured to acquire imagery to the patient. The information relating to the patient can then comprise imagery of the face of the patient and/or of the body of the patient.

According to an example, the at least one pain sensing device comprises a microphone. The information relating to the patient can then comprise one or more sounds associated with the patient.

According to an example, the microphone is integrated into the sheet. Fig. 2 shows a method 100 for displaying patient pain information in its basic steps. The method 100 comprises:
in a using step 110, also referred to as step a), using an input unit to input information relating to at least one known pain area of at least one part of a patient's body;
in a providing step 120, also referred to as step b), providing a processing unit with the information relating to the at least one known pain area of at least one part of the patient's body;
in a laying step 130, also referred to as step c), laying a patient on a sheet, wherein the sheet is configured to support a patient during transfer from a bed of the patient to a bed of a diagnostic imaging system;
in a controlling step 140, also referred to as step d), controlling by the processing unit at least one of at least one light source to provide light illumination at at least one first position of the sheet, wherein control of the at least one of the at last one light source comprises utilization of the information relating to the at least one known pain area of at least one part of the patient's body.

In an example, the at least one light source is integrated into the sheet.

In an example, at least one pressure sensor is integrated into the sheet, and wherein the method comprises providing the processing unit with pressure related information resulting from the patient's body lying on the sheet.

In an example, the method comprises the processing unit utilizing the pressure related information to control the at least one of the at least one light source to provide light illumination at the at least one first position of the sheet.

In an example, the method comprises the processing unit utilizing the pressure related information to control one or more of the at least one light source to provide light illumination at at least one second position of the sheet.

In an example, the method comprises the processing unit processing the pressure related information to determine a change in pressure at a location of the sheet associated with a part of the patient's body when lying on the sheet, The method then comprises the processing unit controlling the one or more of the at least one light source to provide light illumination at the location of the sheet where there was a pressure change.

In an example, the method comprises the processing unit controlling the at least one of the at least light source to provide light at the at least one first position in a first colour and controlling the one or more of the at least one light source to provide light at the at least one second position in a second colour.

In an example, all of the input unit, the processing unit and the at least one light source are separate from the sheet.

In an example, the method comprises the processing unit controlling the at least one light source on the basis of the pain information provided from at least one pain sensing device.

In an example, the at least one pain sensing device comprises a galvanic skin conductance sensor, and wherein the information relating to the patient can comprise a skin conductance of the patient.

In an example, the galvanic skin conductance sensor is at least partially integrated into the sheet.

In an example, the at least one pain sensing device comprises a camera configured to acquire imagery to the patient. The information relating to the patient can comprise imagery of the face of the patient and/or of the body of the patient.

In an example, the at least one pain sensing device comprises microphone. The information relating to the patient can comprise one or more sounds associated with the patient.

In an example, the microphone is integrated into the sheet.

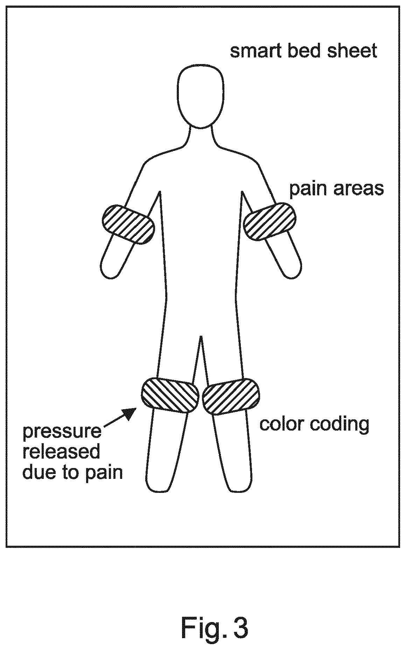

The apparatus and method for displaying patient information are now described in more detail with reference to Fig. 3.

As described above, the normal method of moving a patient from their bed to a scanner such as CT, MR, or PET involves moving the patient while on a bed sheet, with several staff members required to be present and to work together to transfer the patient. This transfer may be painful or at least uncomfortable for the patient. Some patients, for example those handicapped in particular ways or children or through some other reason can find it difficult to communicate that they are in pain, and where they are feeling. Also, staff members who know about where the patient is feeling pain may not accompany the patient during the transfer of the patient to a scanner. The apparatus and method for displaying pain information of a patient, as described above with respect to figs. 1-2, and now described with reference to fig. 3 addresses these issues, representing the pain areas for the patient autonomously even if they cannot express where they are feeling pain verbally.

This invention reduces the movement of the patient during the scan due to external pain. This is visually indicated with the smart bed sheet with colour coding. Reference here to a "sheet" means any thin flexible object, comprising any of material, plastic, rubber, glass fiber, metal (wire or sheet) etc, that is suitable for transferring a patient.

In summary a smart bed sheet is used, which can be helpful to transfer patient to the scan bed and retain the pain information of the patient autonomously. This involves identifying the pain areas for the patient and transferring this pain information to the bed sheet.

Thus, in a particular embodiment the following applies:
- Pre program the pain areas in the bed sheet based on the prior information collected from the patient;
- Pressure sensitive fabrics are used to emphasize the live pain areas of the patient. A pillow/cushion can then be used to release the pressure for corrective action;
- By combining above information, the pain areas can be shown in different colour patches based on the intensity with LEDs embedded in the cloth of bed sheet. This avoids motion of the patient during the scan, the patient can be placed in the correct posture for the scan by taking corrective action for the pain areas;
- Use flexible cloth to change the color or pattern.

Fig. 3 shows a detailed example of one embodiment of the apparatus. A smart bed sheet with embedded LEDs. Existing pain information for the patient is transferred to the smart bed sheet to display the pain areas. This helps the staff to recognize the pain areas of the patient easily with colour coding during the transfer from patient bed to the scanner bed. The information of the pain areas may be gathered by manual input of the patient or the hospital staff via a dedicated user interface that can transfer the information to the bed sheet. The user interface can be displayed on a tablet, notebook or any other computer and may present a whole-body picture or at least parts of the body which can be selected as pain areas. In addition, the intensity of the pain can be indicated, e.g. by a scale from 1 to 10.

In addition to having LEDs embedded in the smart sheet, there are pressure sensors embedded in the smart sheet. If the patient wishes to express his pain areas, he may release the part of the body so that pressure sensors embedded in the smart bed sheet identifies the pain areas by changing the colour. To reduce the false positives, the initial position of the patient on the bed sheet is acquired along with the information relating to existing pain areas, and the present change of colour can be compared against that reference. The staff can take corrective action with pillows/cushions to release the external pain.

In autonomous scanning situations can be encountered where the patient cannot easily express their pain areas to a Health Care (HC) professional - either because they are unable to or that there is no HC professional available. To help address this situation, a pain sensing device is added to the apparatus. Such a pain sensing device is able to measure acute pain - of the type encountered by the physical movement of an (injured) patient. Suitable pain sensing devices are:
- A device which measures a vital sign which rapidly responds to an acute pain. A suitable vital sign can be the galvanic skin conductance (GSC) of the patient, measured using 2 electrodes and a high impedance amplifier attached to a part of the patients body (fingers, wrist or other suitable body part). The GSC has the advantage that a rapid and large increase of skin conductance is recorded around 1 second after an acute pain event, whereby corrective action can be taken before the patient suffers a prolonged pain. The electrodes can also be integrated into the textile of sheet or pillow. For example if an attachment -such as an elasticated bracelet or similar - were to be used to fix the blanket at certain points of the patients body (to ensure correct alignment of the illuminated patches with the patients body part), the electrodes can be integrated into this attaching device. Ideally the electrodes are attached at a point on the inside of the fastening where there is pressure applied to the patients skin - for example on the inside of an elasticated bracelet attaching the blanket at a wrist or ankle.
- A device which measures another biometric signal indicative of pain, for example a camera device for measuring a facial expression or a cramping or spasm of the body. Such a device will however be less specific than the GSR and not so easily integrated into the textile.

If HC professionals are present (or are called), they can take corrective action with pillows/cushions to release the external pain once alerted by the pain sensor. In a more autonomous system, where staff are not easily available - or alternatively if the patient can only communicate with difficulty - the apparatus can further provide a method for localisation of the pain. Such a localisation approach can involve a sequential (small) movement of the body parts at the expected pain areas (for example with a small inflation or deflation of a support structure). An acute pain signal will again be generated when the body part producing pain is moved. At this point the system can either alert a HC professional to only adjust this part of the body or (preferably) adjust the support structure so that even a small motion of the body part does not induce acute pain.

Acute pain often results in spontaneous reactions and verbal expressions or even outcries. A voice processing unit coupled to a microphone can detect such events as they differ substantially from normal spoken language and may indicate to the staff that immediate help/support is required for the patient, e.g. by a rapidly switching of the colour of the whole bed sheet.

It is also possible for the patient to express his/her pain areas by talking to the voice processing unit naming the part of the body and the pain level, and the LEDs can light up at the appropriate areas.

All of the above help in minimizing the pain for the patient for the scan. The pain areas can be shown in different color patches with different intensity levels through LEDs embedded in the cloth of bed sheet. This avoids patient's motion during the scan as we ensure right posture for the scan by taking corrective action for the pain areas.

However, during use of MRI, any additional wiring or electronics contained in the clothes or bed sheet of the patient can lead to dangerous RF heating and patient burns. Additionally, conductive and even paramagnetic components can lead to a loss of image quality. Conversely, the main magnetic field of the MR system may impair the function of electronics in the textiles. Therefore, an embodiment of the apparatus is suitable for MR use that completely avoids any wires or electronics locally at the patient.

Because this excludes local pain and pressure sensors, the pain information input to the system in this embodiment is primarily based on existing pain area information as described above. This pain information is complemented by a camera and microphone system mounted above the patient support that monitors the facial expression and audio of the patient. Video and audio are used to evaluate the emotional state of the patient while being transferred. A coincidence of motion of a body part (as evaluated by the camera), known pain sensitivity of that body part, and painful facial expression or noises (as evaluated by camera and audio) triggers a signal to stop or slow automatic transfer. Then, a projector above the patient support is used to project color coded light spots to the sheet where those body parts that are known to be pain sensitive are located.

Thus, referring to Fig. 3, LEDs are shown in the sheet on which the patient is positioned and are lit to indicate where the patient has pain, with the area being lit slightly larger than that part of the body. Thus, in Fig. 3, some of the LEDs are actually underneath the patient and would be obscured. However, the MR compatible situation, the patient again is lying on the bed sheet and coloured lights are projected onto the sheet, on which the patient is lying. Thus, the patients body part and the sheet around that area are illuminated.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10) for displaying patient pain information, comprising:
- a sheet (20);
- an input unit (30);
- a processing unit (40);
- at least one light source (50);
wherein, the sheet is configured to support a patient during transfer from a bed of the patient to a bed of a diagnostic imaging system;
wherein, the input unit is configured to receive information relating to at least one known pain area of at least one part of the patient's body;
wherein, the input unit is configured to provide the processing unit with the information relating to the at least one known pain area of at least one part of the patient's body; and
wherein, the processing unit is configured to control the at least one of the at least one light source to provide light illumination at at least one first position of the sheet, wherein control of the at least one of the at least one light source comprises utilization of the information relating to the at least one known pain area of at least one part of the patient's body.

2. Apparatus according to claim 1, wherein the at least one light source is integrated into the sheet.

3. Apparatus according to claim 2, comprising at least one pressure sensor (60) integrated into the sheet, and wherein the at least one pressure sensor is configured to provide the processing unit with pressure related information resulting from the patient's body lying on the sheet.

4. Apparatus according to claim 3, wherein the processing unit is configured to utilize the pressure related information to control the at least one of the at least one light source to provide light illumination at the at least one first position of the sheet.

5. Apparatus according to any of claims 3-4, wherein the processing unit is configured to utilize the pressure related information to control one or more of the at least one light source to provide light illumination at at least one second position of the sheet.

6. Apparatus according to claim 5, wherein the processing unit is configured to process the pressure related information to determine a change in pressure at a location of the sheet associated with a part of the patient's body when lying on the sheet, and wherein the processing unit is configured to control the one or more of the at least one light source to provide light illumination at the location of the sheet where there was a pressure change.

7. Apparatus according to any of claims 5-6, wherein the processing unit is configured to control the at least one of the at least one light source to provide light at the at least one first position in a first colour and to control the one or more of the at least one light source to provide light at the at least one second position in a second colour.

8. Apparatus according to claim 1, wherein all of the input unit, the processing unit and the at least one light source are separate from the sheet.

9. Apparatus according to any of claims 1-8, comprising at least one pain sensing device (70); and wherein the at least one pain sensing device is configured to provide the processing unit with information relating to the patient, and wherein the processing unit is configured to control the at least one light source on the basis of the pain information.

10. Apparatus according to claim 9, wherein the at least one pain sensing device comprises a galvanic skin conductance sensor, and wherein the information relating to the patient comprises a skin conductance of the patient.

11. Apparatus according to claim 10 when not dependent on claim 8, wherein the galvanic skin conductance sensor is at least partially integrated into the sheet.

12. Apparatus according to any of claims 9-11, wherein the at least one pain sensing device comprises a camera configured to acquire imagery to the patient, and wherein the information relating to the patient comprises imagery of the face of the patient and/or of the body of the patient.

13. Apparatus according to any of claims 9-12, wherein the at least one pain sensing device comprises a microphone, and wherein the information relating to the patient comprises one or more sounds associated with the patient.

14. Apparatus according to claim 13 when not dependent on claim 8, wherein the microphone is integrated into the sheet.

15. A method (100) for displaying patient pain information, comprising:
a) using (110) an input unit to input information relating to at least one known pain area of at least one part of a patient's body;
b) providing (120) a processing unit with the information relating to the at least one known pain area of at least one part of the patient's body;
c) laying (130) a patient on a sheet, wherein the sheet is configured to support a patient during transfer from a bed of the patient to a bed of a diagnostic imaging system;
d) controlling (140) by the processing unit at least one of at least one light source to provide light illumination at at least one first position of the sheet, wherein control of the at least one of the at last one light source comprises utilization of the information relating to the at least one known pain area of at least one part of the patient's body.
